# EUROPÄISCHE PATENTSCHRIFT

(11) **EP 1 613 227 B1**
(45) Veröffentlichungstag und Bekanntmachung des Hinweises auf die Patenterteilung: **02.12.2009**
(21) Anmeldenummer: 04709210.1
(22) Anmeldetag: 09.02.2004
(51) Int. Cl.: A61B 17/80

(54) **OSTEOSYNTHESEPLATTE ZUR OPERATIVEN VERSORGUNG VON KNOCHENFRAKTUREN**
OSTEOSYNTHESIS PLATE FOR OPERATIVE CARE OF BONE FRACTURES
PLAQUE D'OSTEOSYNTHESE DESTINEE AU TRAITEMENT OPERATOIRE DE FRACTURES OSSEUSES

(30) Priorität: 11.04.2003 DE 10316837; 06.05.2003 DE 10320124
(43) Veröffentlichungstag der Anmeldung: 11.01.2006
(73) Patentinhaber: Königsee Implantate und Instrumente zur Ostheosynthese GmbH, 07426 Königsee (DE)
(72) Erfinder: THIELKE, Karl-Heinz, 32457 Porta Westfalica (DE); BUSCH, Thomas, 07422 Bad Blankenburg (DE); EHRHARDT, Arnd, 07426 Dörnfeld (DE)
(74) Vertreter: Kruspig, Volkmar
(86) Internationale Anmeldenummer: PCT/EP2004/001178
(87) Internationale Veröffentlichungsnummer: WO 2004/089233

(56) Entgegenhaltungen:
- WO-A-01/19267
- WO-A-96/29948
- WO-A-02/096309
- DE-U- 20 022 673
- US-A- 4 493 317
- US-A- 6 096 040
- US-A1- 2002 045 901

## Beschreibung

Die Erfindung betrifft eine Osteosyntheseplatte, insbesondere winkelstabile Radiusplatte, zur operativen Versorgung von Knochenfrakturen, umfassend Bohrungen zur Aufnahme von Knochenschrauben, wobei mindestens einige der Bohrungen mit einem Innengewinde versehen sind, gemäß Oberbegriff des Patentanspruchs 1.

Bei dem Implantat zur Stabilisierung einer Fraktur gemäß DE 197 50 493 A1 ist eine Platte vorgesehen, die Führungen für vorzugsweise zwei Schrauben aufweist, die die Schrauben stabil in einem Winkel zur Platte halten. Die vorerwähnten Führungen sollen es erlauben, die Schrauben auch dann in einem fixierten Winkel zur Platte zu halten, wenn diese sich im Knochen lösen oder sich der Abstand zwischen Platte und Knochen verändert.

Bei der Verwendung von zwei Schrauben, deren Kopf ein konisches Außengewinde besitzt, soll demnach nicht nur eine winkelstabile Fixierung von Kopf- und Schaftfragment des Knochens zueinander gewährleistet werden, sondern darüber hinaus noch eine zusätzliche Rotationsstabilität erzielt werden, welche ein Auslockern dieser Schrauben, z. B. bei Pendelübungen verhindert. Der von den Führungen vorgegebene Winkel entspricht gemäß DE 197 50 493 A1 in etwa dem physiologischen Winkel des Kopfes und liegt im Bereich zwischen 35° und 40°, gemessen zur Längsachse des Schafts. Die Platte besitzt weiterhin in ihrem unteren Teil auf einer durchgehenden Linie drei Bohrungen, von denen eine mittlere Bohrung als Langloch ausgeführt ist. Diese Bohrungen dienen zur Aufnahme üblicher Kortikalisschrauben. Die Auflagefläche der Platte selbst ist konkav ausgeführt, wodurch die Platte besser auf der Oberfläche des Knochens zum Anliegen kommen soll. Durch die mittlere Bohrung als Langloch besteht die Möglichkeit, eine günstige Position für eine entsprechende Kortikalisschraube bezogen auf die Knochensubstanz zu finden. Dies wäre an sich auch für die weiteren Bohrungen wünschenswert, jedoch tritt dann ebenfalls der vorstehend beschriebene Nachteil der unerwünschten Materialschwächung ein, der noch dadurch verstärkt wird, dass die Platte von oben betrachtet seitliche Einschnürungen aufweist.

Aus der DE 43 41 980 A1 ist eine osteosynthetische Knochenplatte vorbekannt, die mehrere in Längsrichtung angeordnete runde und/oder längliche Löcher zur Aufnahme von Knochenschrauben besitzt.

Die in der Knochenplatte angeordneten Löcher weisen ein konisches Gewinde auf, wobei der Schraubenkopf der Knochenschrauben ebenso ein konisches, jedoch Außengewinde besitzt. Durch die Gestaltung der Löcher in der Knochenplatte und des Schraubenkopfes der Knochenschraube mit einem konischen Gewinde wird erreicht, dass sich der Schraubenkopf beim Anziehen in der Knochenplatte verklemmt und die Knochenschrauben auf diese Weise fest mit der Knochenplatte in Verbindung stehen. Als Vorteil wird dort herausgestellt, dass die sichere Verbindung der Knochenbruchstücke nicht durch ein Anpressen der Knochenplatte auf die Knochenoberfläche, sondern nur durch den Sitz der Knochenschrauben in den Knochenbruchstücken erreicht wird, so dass die Knochenhaut nicht zusätzlich beeinträchtigt ist und sich auf diese Weise der Heilungsprozess beschleunigt.

Mindestes ein Loch der aus der DE 43 41 980 A1 bekannten Platte weist parallel zur Plattenlängsachse verlaufend eine längliche Form auf, wobei die endseitigen Radien unterschiedliche Abmessungen besitzen.

Der zur Knochenbruchstelle abgewandte Radius ist kleiner gestaltet als der zur Knochenbruchstelle zugewandte Radius. Das längliche Loch ist konisch ausgeführt und mit einem umlaufenden Innengewinde versehen. Die Konizität dieses Loches entspricht wiederum der des Schraubenkopfes einer entsprechenden Knochenschraube. Beim Festziehen der Knochenschraube stellt sich dann eine Bewegung längs der Knochenplatte und damit eine Kompression der Knochenbruchstücke ein. Ein enges Besetzen eines Knochenstücks mit mehreren Schrauben, gegebenenfalls auch kleineren Durchmessers, ist nach der DE 43 41 980 A1 nicht möglich. Wären mehrere der eine Längsform aufweisenden Löcher benachbart, so ergeben sich Stabilitätsprobleme durch Schwächung des Plattenmaterials. Dies könnte wiederum nur durch eine größere Dicke ausgeglichen werden, so dass sich der Aufbau insgesamt in unerwünschter Weise durch die größere Plattenstärke erhöht.

Bei der Osteosyntheseplatte nach EP 0 468 192 A2 sind mehrere, die Oberseite und die Unterseite der Platte verbindende Bohrungen zur Aufnahme von Knochenschrauben vorgesehen.

Gemäß der dort als bevorzugte Ausführungsform dargestellten Lösung sind die Plattenlöcher konisch ausgebildet und vorzugsweise mit einem Innengewinde versehen. Die zur Fixierung der Platte verwendeten Schrauben weisen einen konisch auslaufenden Kopf, vorzugsweise mit konischem Außengewinde auf. Die Schrauben werden durch die Plattenlöcher hindurch in an sich bekannter Weise in den Knochen eingedreht. Bei vollständigem Eindrehen der Schraube verspannt sich der konische Schraubenkopf in der konischen Plattenbohrung, wobei dieser Effekt durch die vorzugsweise eingebrachten Gewindegänge unterstützt wird. Dieses vorerwähnte Verspannen ist dann wesentlich, wenn die Schraube nur unikortal eingesetzt werden soll und die Platte nicht auf der Knochenoberfläche aufliegt. Die konische Schraubenverbindung ist insofern vorteilhaft, da sich die Gewindegänge bei festem Anziehen ineinander verkeilen. Dieses Verkeilen vermindert die Gefahr einer unbeabsichtigten Lockerung der Platten/Schraubenverbindung bei entsprechenden wiederkehrenden Belastungen.

Gemäß WO 02/096309 A1 ist eine Knochenplatte bekannt, welche im stilförmigen Teil der Platte mehrere Schraubenlöcher mit Innengewinden zur Aufnahme von Knochenschrauben aufweist, wobei einzelne Schraubenlöcher aus mindestens zwei Bohrungen bestehen, welche teilweise ineinander greifend und überlappend ausgeführt sind.

Aus dem Vorgenannten ist es daher Aufgabe der Erfindung, eine weiterentwickelte Osteosyntheseplatte, insbesondere winkelstabile Radiusplatte, zur operativen Versorgung von Knochenfrakturen anzugeben, welche es ermöglicht, wesentlich größere Freiheitsgrade beim Besetzen des Knochens zu erhalten, und wobei die Gefahr von Knochenrissen beim Eintreiben der Knochenschrauben weitgehend reduziert ist und eine ausreichende Stabilität der Osteosyntheseplatte gewährleistet ist.

Die Lösung der Aufgabe der Erfindung erfolgt mit einer Osteosyntheseplatte, insbesondere winkelstabilen Radiusplatte, gemäß den Merkmalen des Patentanspruchs 1, wobei die Unteransprüche zweckmäßige Ausgestaltungen und Weiterbildungen umfassen.

Demnach sind erfindungsgemäß in der Osteosyntheseplatte mindestens zwei Bohrungen teilweise ineinander greifend und überlappend ausgeführt, wobei die jeweils ineinander greifenden Bohrungen in Plattenlängs- und/oder -querrichtung, insbesondere aber auch auf einem vorgegebenen Radius angeordnet werden können und zwischen zwei beabstandeten Bohrungen größeren Durchmessers eine diese schneidende Bohrung kleineren Durchmessers ausgebildet ist. Die ineinander greifenden Bohrungen weisen jeweils ein konisches Gewinde auf.

Mit einer solchen Abfolge von einer Bohrung größeren Durchmessers, einer Bohrung kleineren Durchmessers und wiederum einer Bohrung größeren Durchmessers ist einer unerwünschten Reduzierung der Stabilität der Platte über ihre relevante Quer- oder Längsschnittfläche wirksam entgegengetreten.

Der Bohrungsdurchmesser kann bezogen auf übliche Bohrungen nach dem Stand der Technik reduziert werden, da durch das mögliche redundante Setzen mehrerer Knochenschrauben in die jeweiligen Bohrungsgruppen für die notwendige Stabilität insgesamt Sorge getragen ist. Andererseits besteht die Möglichkeit, bezogen auf die Lage einer Fraktur eine der Bohrungen auszuwählen, ohne dass die Gefahr besteht, dass im Bereich oder in der Nähe der Fraktur ein Knochenriss auftritt.

Bei einer Ausführungsform der Erfindung sind die Bohrungswinkel, d.h. die Bohrungslängsachsen, nicht rechtwinklig zum Plattenlängs- oder Plattenquerschnitt ausgeführt, sondern in einem vom rechten Winkel abweichenden Maß, so dass definierte Schrägstellungen der Knochenschrauben beim Eintreiben dieser erreicht werden können.

Bevorzugt liegt der jeweilige Bohrungswinkel bezogen auf die Plattenlängsachse im Bereich von im wesentlichen 45° bis 135°. Dabei können benachbarte Bohrungen der ineinander greifenden Bohrungsgruppen unterschiedliche Winkel mit unterschiedlichen Schrägstellungen eingedrehter Knochenschrauben besitzen. Durch diese Maßnahme wird wie vorerwähnt die Gefahr von unerwünschten Knochenrissen oder eine Ausdehnung der Fraktur bei der Befestigung der Osteosyntheseplatte verringert.

Die erfindungsgemäßen Überlappungen der ineinander greifenden Bohrungen liegen im Bereich von 10% bis 35% des durchschnittlichen Bohrungsdurchmessers einer Einzelbohrung.

Bei einer weiteren Ausführungsform wird davon ausgegangen, dass der Durchmesser der ineinander greifenden, überlappenden Bohrungen unterschiedliche Werte annimmt.

Bei einer Ausführungsform der Erfindung besitzt der Plattenquerschnitt eine Sichelform. Dabei zeigt der konvexe Teil der Sichel nach oben, wobei der konkave Sichelteil zur Knochenoberfläche gerichtet ist. Erfindungsgemäß ist der konvexe Teil der Sichel mit einem größeren Radius ausgeführt, als dies im konkaven, unteren Teil der Platte der Fall ist.

Die einzusetzenden Knochenschrauben weisen bevorzugt ein zu den Gewindebohrungen komplementäres konisches Gewinde auf, so dass der aus dem Stand der Technik bekannte Effekt des Verklemmens mit der Folge eines sicheren winkelstabilen Halts der Schrauben eintritt.

Ausgestaltend weisen die Schraubenköpfe eine Kugelkopf-Gewindeform auf. In diesem Fall besitzen die Gewindebohrungen mindestens in ihrem oberen Abschnitt eine zu den Schraubenköpfen komplementäre Kugelkopfform.

Bei der winkelstabilen Radiusplatte ist vorgesehen, dass die Platte einen breiteren Plattenkopf aufweist, wobei im Plattenkopf zwischen zwei beabstandeten Bohrungen die mindestens zwei ineinander greifenden Bohrungen mit konischem Innengewinde angeordnet sind.

Es können die beabstandeten Bohrungen sowie die ineinander greifenden Bohrungen erfindungsgemäß auf einem gemeinsamen Radius liegen. Ergänzend besteht die Möglichkeit, im Kopfbereich mindestens ein zusätzliches Langloch vorzusehen, welches sich im Übergang zwischen Kopf und Plattenlängsbereich befindet.

Mindestens ein weiteres Langloch kann sich parallel zur Längsachse der Platte erstrecken.

Optional kann der Plattenkopf eine Wölbung aufweisen. Zwischen dem Plattenkopf und dem Plattenlängsbereich ist eine Kröpfung ausgebildet, so dass ein anatomisch angeglichenes Implantat entsteht. Im Übergang zwischen Plattenlängsbereich und Plattenkopf weist das Implantat eine sich kontinuierlich vergrößernde Breite auf. Der Plattenkopf selbst kann eine asymmetrische Form besitzen, wobei außenseitig als Umschreibende diese Form ein ungleichseitiges Dreieck bildet.

Das freie Ende des Plattenlängsbereichs weist in einer bevorzugten Ausgestaltung einen Rundungsradius auf. Die Bohrungen zur Aufnahme der Knochenschrauben sind versenkt ausgeführt, wodurch bei eingeschraubten Knochenschrauben eine im wesentlichen bündige Oberfläche resultiert.

Die Erfindung soll nachstehend anhand eines Ausführungsbeispiels sowie unter Zuhilfenahme von Figuren näher erläutert werden.

Hierbei zeigen:
- Fig. 1: Seitenansicht und Draufsichten auf eine Radiusplatte gemäß erster Ausführungsform der Erfindung sowie eine Schnittdarstellung des Details der ineinander greifenden, überlappenden Bohrungen;
- Fig. 2: ein weiteres Ausführungsbeispiel einer winkelstabilen Radiusplatte, links, mit Langloch im Kopfbereich sowie umfassend Detaildarstel- lungen und einen Schnitt längs der Linie A-A;
- Fig. 3: ein Ausführungsbeispiel einer Radiusplatte, ebenfalls in winkelstabi- ler Ausführung, rechts, mit ergänzenden Bohrungen im Übergangs- bereich des Kopfteils sowie Detaildarstellungen und einen Schnitt längs der Linie A-A, sowie
- Fig. 4: ein Ausführungsbeispiel einer winkelstabilen Radiusplatte, links, ana- log der Darstellung nach Fig. 3, ebenfalls umfassend Detailabbildun- gen sowie einen Schnitt längs der Linie A-A.

Die Osteosyntheseplatte nach den Figuren geht von einem Plattenlängsbereich 1, einem gekröpften Übergangsbereich 2 sowie einem Plattenkopf 3 aus.

Im Plattenlängsbereich befinden sich im wesentlichen auf der Längsachse verlaufende Bohrungen 4, die zur Aufnahme mindestens eines Teiles eines Schraubenkopfes (nicht gezeigt) versenkt ausgeführt sind.

Je nach der Länge der Platte, z. B. im Bereich von 51 mm bis 93 mm, variiert die Lochzahl zwischen drei und acht.

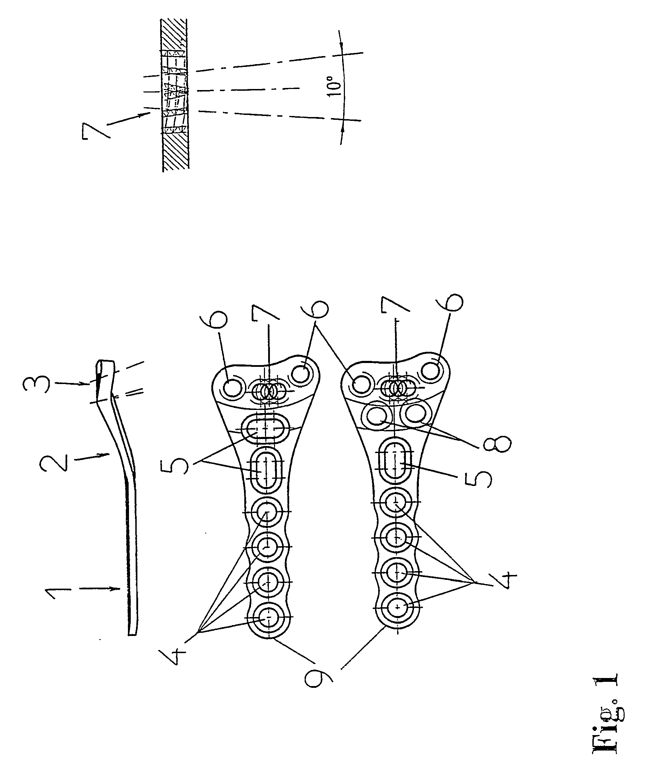

Bei der Ausführungsform der Platte nach Fig. 1, Mitte, sind zwei beabstandete, senkrecht zueinander orientierte Langlöcher 5 im gekröpften Übergangsbereich ausgebildet.

Zwischen zwei Kopfbohrungen 6 befindet sich ein Abschnitt mit mehreren ineinander greifenden Bohrungen 7.

Diese ineinander greifenden Bohrungen 7 besitzen ein konisches Gewinde und können zueinander unter einem Winkel stehen.

Beim Ausführungsbeispiel gemäß Fig. 1, untere Darstellung, ist anstelle des querverlaufenden Langlochs 5 die Ausbildung von zwei weiteren Kopfbohrungen 8 vorgesehen.

Der Übergang zwischen Plattenlängsbereich 1 und Plattenkopf 3 verläuft breitenseitig stetig ansteigend.

Die Platte ist insbesondere im Bereich des Kopfes 3 anatomisch geformt.

Bei den Ausführungsbeispielen nach den Figuren wird von einem Dreifach-Bohrungspaar im Plattenkopf 3 ausgegangen, jedoch sind auch Zweifach-Bohrungspaare oder Bohrungspaare mit einer Anzahl größer drei, je nach Verwendung und Plattengröße, denkbar.

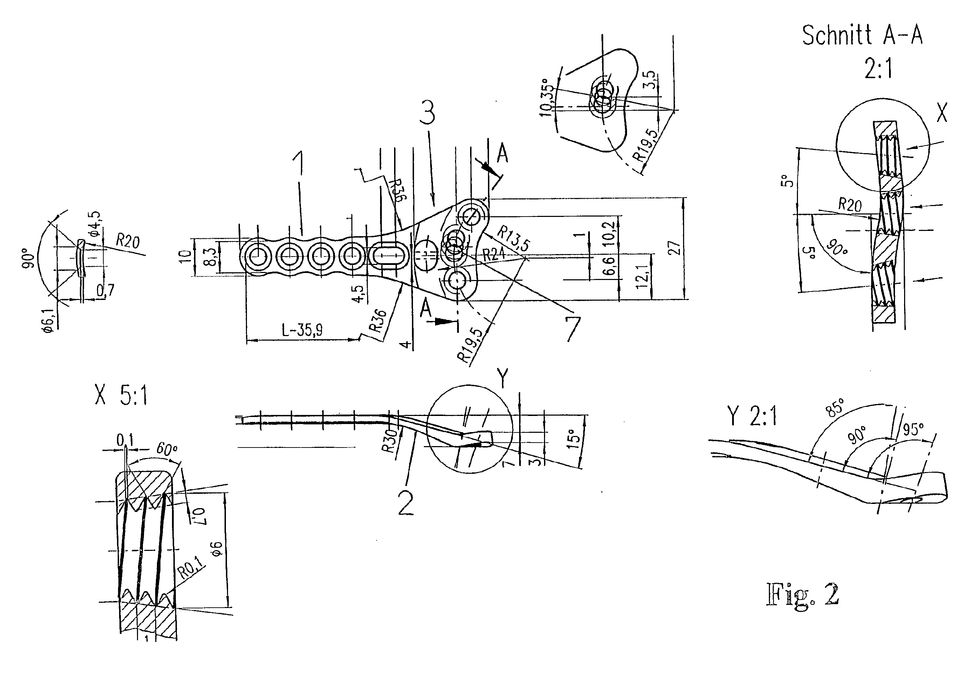

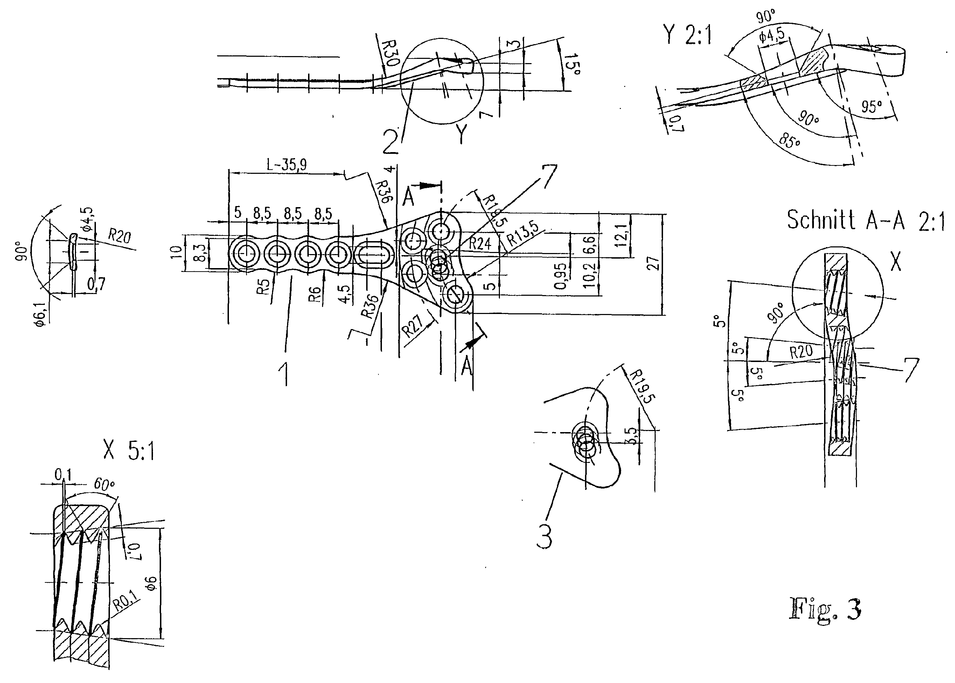

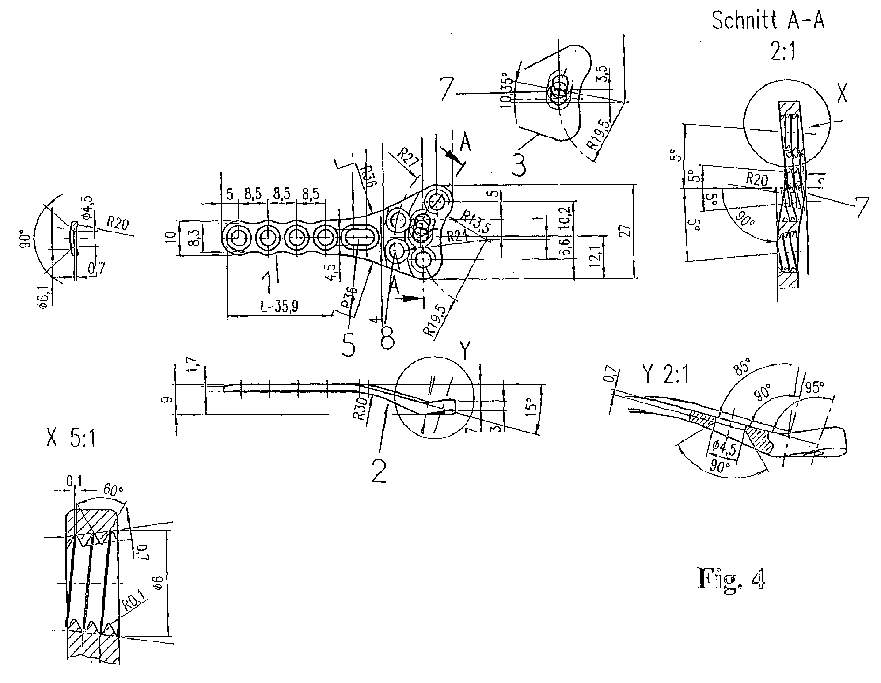

Bei den Darstellungen nach Fig. 1 befinden sich die ineinander greifenden Bohrungen 7 auf einer gedachten geraden Linie; hingegen bei den Fig. 2 bis 4 auf einem Radius, auf dem auch die äußeren Kopfbohrungen 6 befindlich sein können.

Die Vielzahl der ineinander greifenden Bohrungen gestattet ein vorteilhaftes engeres Besetzen, was wiederum zur Folge hat, dass Knochenschrauben geringeren Durchmessers eingesetzt werden können. Andererseits kann aber der Operateur auch eine der relevanten Bohrungen, die der Fraktur entfernt liegt, benutzen, so dass korrigierende Maßnahme am Implantat nicht vorgenommen werden müssen.

Der Vorteil der Langlöcher 5 liegt darin, dass beim Eintreiben einer Knochenschraube im Bereich des Langlochs noch eine Winkelanpassung bzw. Justage des Implantats möglich ist. Auch kann durch das jeweilige Langloch Knochensubstanz in den Frakturbereich eingebracht werden.

Die jeweiligen Bohrungswinkel der ineinander greifenden Bohrungen können bezogen auf die Plattenlängs- oder aber Plattenquerachse variieren und verlaufen bevorzugt im wesentlichen zwischen 45° und 135°. Hier wiederum ist es von Vorteil, den Bohrungswinkel benachbarter Bohrungen voneinander abweichend, insbesondere ausgehend von einer gedachten Lotrechten, unter einem Winkel von z. B. +35° für die eine Bohrung und -35° für die benachbarte Bohrung auszuführen.

Der Überlappungsbereich der ineinander greifenden Bohrungen liegt bei etwa 10% bis 35% des durchschnittlichen Bohrungsdurchmessers, so dass die Eigenstabilität je Bohrung beim Einbringen einer zugehörigen Knochenschraube erhalten bleibt.

Aus den Figuren wird weiterhin deutlich, dass der Plattenkopf 3 unter Beachtung der anatomischen Verhältnisse gewölbt ausführbar ist.

Die Form des Plattenkopfes kann im übrigen asymmetrisch gewählt werden, wobei außenseitig als Umschreibende die Form des Plattenkopfes der eines ungleichseitigen Dreiecks nahe kommt.

Das freie Ende des Plattenlängsbereichs 1 weist einen Rundungsradius 9 auf.

Alles in allem stellt die vorgestellte Osteosyntheseplatte, insbesondere winkelstabile Radiusplatte, ein anatomisch angeglichenes Implantat, z. B. für die körperferne Speiche an deren Palmarseite dar, wobei während des eigentlichen Operationsvorgangs und zu dessen Ausführung keine korrigierenden Maßnahmen am Implantat vorgenommen werden müssen. Die ineinander greifenden Bohrungen gestatten hier eine große Flexibilität bei der Anwendung des Implantats, ohne dass die Stabilitätseigenschaften der Platte in nachhaltiger Weise beeinflusst werden.

Im Gegensatz zu ansonsten üblichen ausschließlichen Langlochbohrungen sind bei ineinander greifenden, überlappenden Bohrungen mit entsprechendem Gewinde mehrere Schrauben exakt oder wahlweise positioniert und winkelstabil einbringbar, mit dem weiteren Vorteil der jeweils voneinander abweichenden Winkelorientierung der Bohrung und der dem Bohrungsverlauf folgenden Knochenschraube.

### Bezugszeichenliste

- 1: Plattenlängsbereich
- 2: gekröpfter Bereich
- 3: Plattenkopf
- 4: Bohrungen
- 5: Langloch
- 6: Kopfbohrung
- 7: ineinander greifende Bohrungen
- 8: weitere Kopfbohrung
- 9: Rundungsradius

## Patentansprüche

1. Osteosyntheseplatte, insbesondere winkelstabile Radiusplatte, zur operativen Versorgung von Knochenfrakturen, umfassend Bohrungen (4, 5, 6, 7) zur Aufnahme von Knochenschrauben, wobei mindestens einige der Bohrungen mit einem Innengewinde versehen sind, weiterhin mindestens zwei Bohrungen (7) teilweise ineinander greifend und überlappend ausgeführt sind, wobei die jeweils ineinander greifenden Bohrungen in Plattenlängs- und/oder -querrichtung oder auf einem vorgegebenen Radius angeordnet sind,
**dadurch gekennzeichnet, dass**
zwischen zwei beabstandeten Bohrungen größeren Durchmessers eine diese schneidende Bohrung kleineren Durchmessers vorgesehen ist und die ineinander greifenden Bohrungen jeweils ein konisches Gewinde aufweisen.

2. Osteosyntheseplatte nach Anspruch 1,
**dadurch gekennzeichnet, dass**
der jeweilige Bohrungswinkel bezogen auf die Plattenlängsachse im Bereich von im wesentlichen 45° bis 135° liegt.

3. Osteosyntheseplatte nach Anspruch 1,
**dadurch gekennzeichnet, dass**
der jeweilige Bohrungswinkel bezogen auf die Plattenquerachse im Bereich von im wesentlichen 45° bis 135° liegt.

4. Osteosyntheseplatte nach einem der vorangegangenen Ansprüche,
**dadurch gekennzeichnet, dass**
jede der ineinander greifenden Bohrungen einen unterschiedlichen Bohrungswinkel aufweist.

5. Osteosyntheseplatte nach einem der vorangegangenen Ansprüche,
**dadurch gekennzeichnet, dass**
die Überlappung der ineinander greifenden Bohrungen im Bereich zwischen 10% und 35% des durchschnittlichen Bohrungsdurchmessers liegt.

6. Osteosyntheseplatte nach einem der vorangegangenen Ansprüche,
**dadurch gekennzeichnet, dass**
der Plattenquerschnitt eine Sichelform aufweist.

7. Osteosyntheseplatte nach einem der vorangegangenen Ansprüche,
**dadurch gekennzeichnet, dass**
die einzusetzenden Knochenschrauben ein zu den Gewindebohrungen komplementäres konisches Gewinde besitzen.

8. Osteosyntheseplatte nach einem der vorangegangenen Ansprüche,
**dadurch gekennzeichnet, dass**
die Schraubenköpfe eine Kugelkopf-Gewindeform aufweisen.

9. Osteosyntheseplatte nach Anspruch 9,
**dadurch gekennzeichnet, dass**
die Gewindebohrungen mindestens in ihrem oberen Abschnitt eine zu den Schraubenköpfen komplementäre Kugelkopfform besitzen.

10. Osteosyntheseplatte nach einem der vorangegangenen Ansprüche,
**dadurch gekennzeichnet, dass**
die Platte einen breiteren Plattenkopf aufweist, wobei im Plattenkopf zwischen zwei beabstandeten Bohrungen die mindestens zwei ineinander greifenden Bohrungen mit konischem Innengewinde angeordnet sind.

11. Osteosyntheseplatte nach Anspruch 11,
**dadurch gekennzeichnet, dass**
die beabstandeten Bohrungen sowie die ineinander greifenden Bohrungen auf einem gemeinsamen Radius liegen.

12. Osteosyntheseplatte nach Anspruch 11 oder 12,
**dadurch gekennzeichnet, dass**
im Kopfbereich zusätzlich mindestens ein Langloch vorgesehen ist, welches sich im Übergang zwischen Kopf und Plattenlängsbereich befindet.

13. Osteosyntheseplatte nach Anspruch 13,
**dadurch gekennzeichnet, dass**
mindestens ein Langloch sich parallel zur Längsachse der Platte erstreckt.

14. Osteosyntheseplatte nach Anspruch 13,
**dadurch gekennzeichnet, dass**
mindestens ein Langloch sich senkrecht zur Längsachse der Platte erstreckt.

15. Osteosyntheseplatte nach einem der Ansprüche 11 bis 15,
**dadurch gekennzeichnet, dass**
der Plattenkopf gewölbt ist.

16. Osteosyntheseplatte nach einem der Ansprüche 11 bis 16,
**dadurch gekennzeichnet, dass**
zwischen Plattenkopf und Plattenlängsbereich eine Kröpfung ausgebildet ist.

17. Osteosyntheseplatte nach einem der Ansprüche 11 bis 17,
**dadurch gekennzeichnet, dass**
diese im Übergang zwischen Plattenlängsbereich und Plattenkopf eine sich kontinuierlich vergrößernde Breite aufweist.

18. Osteosyntheseplatte nach einem der Ansprüche 11 bis 18,
**gekennzeichnet durch**
einen asymmetrischen Plattenkopf.

19. Osteosyntheseplatte nach Anspruch 19,
**dadurch gekennzeichnet, dass**
der Plattenkopf außenseitig als Umschreibende die Form eines ungleichseitigen Dreiecks aufweist.

20. Osteosyntheseplatte nach einem der vorangegangenen Ansprüche,
**dadurch gekennzeichnet, dass**
das freie Ende des Plattenlängsbereichs einen Rundungsradius aufweist.

21. Osteosyntheseplatte nach einem der vorangegangenen Ansprüche,
**dadurch gekennzeichnet, dass**
alle Bohrungen zur Aufnahme von Knochenschrauben versenkt ausgebildet sind.

## Claims

1. An osteosynthesis plate, in particular an angle-stable radius plate, for the operative care of bone fractures, comprising holes (4, 5, 6, 7) for the insertion of bone screws, with at least some of the holes being provided with a female thread, further at least two holes (7) being formed partially engaging and overlapping, with each of the engaging holes being arranged in the longitudinal and/or transverse direction of the plate or on a pregiven radius,
**characterised in that**
between two spaced holes with a larger diameter, one hole is provided with a smaller diameter, which intersects said holes, and the engaging holes each comprise a conical thread.

2. The osteosynthesis plate according to Claim 1,
**characterised in that**
the respective hole angle, relative to the longitudinal axis of the plate, is in the range from essentially 45° to 135°.

3. The osteosynthesis plate according to Claim 1,
**characterised in that**
the respective hole angle, relative to the transverse axis of the plate, is in the range from essentially 45° to 135°.

4. The osteosynthesis plate according to one of the previous claims,
**characterised in that**
each of the engaging holes has a different hole angle.

5. The osteosynthesis plate according to one of the previous claims,
**characterised in that**
the overlap of the engaging holes is within the range from 10% to 35% of the average hole diameter.

6. The osteosynthesis plate according to one of the previous claims,
**characterised in that**
the plate cross-section is crescent-shaped.

7. The osteosynthesis plate according to one of the previous claims,
**characterised in that**
the bone screws to be inserted have a conical thread which is complementary to the threaded holes.

8. The osteosynthesis plate according to one of the previous claims,
**characterised in that**
the screw heads have a spherical head thread shape.

9. The osteosynthesis plate according to Claim 8,
**characterised in that**
the threaded holes have a complementary spherical head shape at least in their upper portion, which matches the screw heads.

10. The osteosynthesis plate according to one of the previous claims,
**characterised in that**
the plate comprises a wider plate head, with the at least two engaging holes with conical female thread being arranged in the plate head between two spaced holes.

11. The osteosynthesis plate according to Claim 10,
**characterised in that**
the spaced holes as well as the engaging holes lie on a common radius.

12. The osteosynthesis plate according to Claim 10 or 11,
**characterised in that**
the at least one elongated hole is additionally provided in the head area, which is located in the transition between the head and the longitudinal area of the plate.

13. The osteosynthesis plate according to Claim 12,
**characterised in that**
at least one elongated hole extends parallel to the longitudinal axis of the plate.

14. The osteosynthesis plate according to Claim 12,
**characterised in that**
at least one elongated hole extends vertically to the longitudinal axis of the plate.

15. The osteosynthesis plate according to one of Claims 10 to 14,
**characterised in that**
the plate head is dome-shaped.

16. The osteosynthesis plate according to one of Claims 10 to 15,
**characterised in that**
**that** a bend is formed between the plate head and the longitudinal area of the plate.

17. The osteosynthesis plate according to one of Claim 10 to 16,
**characterised in that**
it comprises a continually increasing width in the transition between the longitudinal area of the plate and the plate head.

18. The osteosynthesis plate according to one of Claims 10 to 17,
**characterised by**
an asymmetric plate head.

19. The osteosynthesis plate according to Claim 18,
**characterised in that**
the plate head on its outside comprises as an envelope the shape of a scalene triangle.

20. The osteosynthesis plate according to one of the previous claims,
**characterised in that**
the free end of the longitudinal plate area comprises a rounding radius.

21. The osteosynthesis plate according to one of the previous claims,
**characterised in that**
all holes for the insertion of bone screws are formed to be countersunk.

## Revendications

1. Plaque d'ostéosynthèse, en particulier plaque de radius présentant un angle stable, pour le traitement opératoire de fractures osseuses, comprenant des perçages (4, 5, 6, 7) pour recevoir des vis à os, dans laquelle au moins quelques-uns des perçages sont dotés d'un taraudage, et au moins deux perçages (7) sont en outre réalisés de manière à s'engager partiellement l'un dans l'autre et à se chevaucher, lesdits perçages qui s'engagent respectivement l'un dans l'autre étant agencés en direction longitudinale et/ou transversale de la plaque ou sur un rayon prédéterminé,
**caractérisée en ce que**
entre deux perçages écartés de grand diamètre il est prévu un perçage de plus petit diamètre qui les recoupe, et les perçages qui s'engagent l'un dans l'autre présentent chacun un pas de vis conique.

2. Plaque d'ostéosynthèse selon la revendication 1,
**caractérisée en ce que** l'angle de perçage respectif, par référence à l'axe longitudinal de la plaque, est dans la plage de sensiblement 45° à 135°.

3. Plaque d'ostéosynthèse selon la revendication 1,
**caractérisée en ce que** l'angle de perçage respectif, par référence à l'axe transversal de la plaque, est dans la plage de sensiblement 45° à 135°.

4. Plaque d'ostéosynthèse selon l'une des revendications précédentes,
**caractérisée en ce que** chacun des perçages qui s'engagent l'un dans l'autre présente un angle de perçage différent.

5. Plaque d'ostéosynthèse selon l'une des revendications précédentes,
**caractérisée en ce que** le chevauchement des perçages qui s'engagent l'un dans l'autre est dans la plage entre 10 % et 35 % du diamètre de perçage moyen.

6. Plaque d'ostéosynthèse selon l'une des revendications précédentes,
**caractérisée en ce que** la section de la plaque a une forme en croissant.

7. Plaque d'ostéosynthèse selon l'une des revendications précédentes,
**caractérisée en ce que** les vis à os à employer possèdent un pas de vis conique complémentaire aux perçages taraudés.

8. Plaque d'ostéosynthèse selon l'une des revendications précédentes,
**caractérisée en ce que** les têtes de vis présentent une forme à pas de vis à tête sphérique.

9. Plaque d'ostéosynthèse selon la revendication 8,
**caractérisée en ce que** les perçages taraudés possèdent, au moins dans leur tronçon supérieur, une forme à tête sphérique complémentaire des têtes des vis.

10. Plaque d'ostéosynthèse selon l'une des revendications précédentes,
**caractérisée en ce que** la plaque présente une tête de plaque plus large, et dans la tête de plaque entre deux perçages écartés sont agencés lesdits au moins deux perçages qui s'engagent l'un dans l'autre avec un taraudage conique.

11. Plaque d'ostéosynthèse selon la revendication 10,
**caractérisée en ce que** les perçages écartés ainsi que les perçages qui s'engagent l'un dans l'autre sont situés sur un rayon commun.

12. Plaque d'ostéosynthèse selon la revendication 10 ou 11,
**caractérisée en ce que** dans la zone de la tête il est prévu en supplément au moins un trou oblong qui se trouve dans la transition entre la tête et la région longitudinale de la plaque.

13. Plaque d'ostéosynthèse selon la revendication 12,
**caractérisée en ce qu'**au moins un trou oblong s'étend parallèlement à l'axe longitudinal de la plaque.

14. Plaque d'ostéosynthèse selon la revendication 12,
**caractérisée en ce qu'**au moins un trou oblong s'étend perpendiculairement à l'axe longitudinal de la plaque.

15. Plaque d'ostéosynthèse selon l'une des revendications 10 à 14,
**caractérisée en ce que** la tête de la plaque est bombée.

16. Plaque d'ostéosynthèse selon l'une des revendications 10 à 15,
**caractérisée en ce qu'**un coude est réalisé entre la tête de la plaque et la région longitudinale de la plaque.

17. Plaque d'ostéosynthèse selon l'une des revendications 10 à 16,
**caractérisée en ce que** celle-ci présente une largeur qui va continuellement en s'agrandissant dans la transition entre la région longitudinale de la plaque et la tête de la plaque.

18. Plaque d'ostéosynthèse selon l'une des revendications 10 à 17,
**caractérisée par** une tête de plaque asymétrique.

19. Plaque d'ostéosynthèse selon la revendication 18,
**caractérisée en ce que** la tête de la plaque présente du côté extérieur une forme que l'on peut décrire comme étant un triangle non-isocèle.

20. Plaque d'ostéosynthèse selon l'une des revendications précédentes,
**caractérisée en ce que** l'extrémité libre de la région longitudinale de la plaque présente un rayon arrondi.

21. Plaque d'ostéosynthèse selon l'une des revendications précédentes,
**caractérisée en ce que** tous les perçages sont réalisés en renfoncement, pour la réception des vis à os.
